# EUROPÄISCHE PATENTSCHRIFT

(11) **EP 0 724 907 B1**
(45) Veröffentlichungstag und Bekanntmachung des Hinweises auf die Patenterteilung: **03.09.1997**
(21) Anmeldenummer: 95120400.7
(22) Anmeldetag: 22.12.1995
(51) Int. Cl.: B01J 23/44, B01J 37/02

(54) **Palladium-Trägerkatalysatoren**
Palladium supported catalysts
Catalyseur sur support au palladium

(30) Priorität: 09.01.1995 DE 19500366
(43) Veröffentlichungstag der Anmeldung: 07.08.1996
(73) Patentinhaber: BASF AKTIENGESELLSCHAFT, 67056 Ludwigshafen (DE)
(72) Erfinder: Heineke, Daniel, Dr., D-67065 Ludwigshafen (DE); Flick, Klemens, Dr., D-76863 Herxheim (DE); Wünsch, Martin, Dr., D-67158 Ellerstadt (DE)

(56) Entgegenhaltungen:
- EP-A- 0 412 415
- EP-A- 0 430 435
- EP-A- 0 503 470
- EP-A- 0 634 214
- US-A- 4 048 092

## Beschreibung

Die vorliegende Erfindung betrifft Palladium-Trägerkatalysatoren mit einem Palladiumgehalt von 0,001 bis 2 Gew.-%, die Palladium, im wesentlichen in Abwesenheit von Promotormetallen, in einer Schichtdicke von weniger als 5000 nm als Schale auf dem Träger enthalten und man das Palladium als Sol durch Tränkung auf einen Träger oder durch Aufsprühen auf einen beheizten Träger aufbringt sowie deren Einsatz zur Hydrierung von Acetylenen und Dienen.

Acetylene und Diene sind aufgrund ihrer Neigung zur Polymerisation oder ihrer ausgeprägten Neigung zur Komplexbildung an Übergangsmetallen für verschiedene technisch wichtige Synthesen unerwünschte Stoffe, da Acetylene und Diene die bei diesen Reaktionen verwendeten Katalysatoren stark beeinträchtigen. So stört z.B. das im C₂-Strom eines Steamcrackers enthaltene Acetylen die Polymerisation des Ethylens, sodaß der Acetylengehalt im C₂-Strom kleiner 1 ppm betragen muß. Auch der C₃-Strom ex Steamcracker, der neben Propylen auch 2 bis 3 % Propadien (PD) und etwa die gleiche Menge Propin (Methylacetylen, MA) enthält, muß vor der Polymerisation zu Polypropylen gereinigt werden. Dabei wird der Gesamt-Gehalt an Acetylenen und Dienen durch Hydrierung unter 20 ppm gedrückt. Beide Kohlenwasserstoffströme werden in der industriellen Praxis durch selektive Hydrierung an heterogenen Edelmetallkatalysatoren auf keramischen Trägern gereinigt. Dabei werden hohe Anforderungen an die verwendeten Hydrierkatalysatoren hinsichtlich ihrer Selektivität und Aktivität gestellt, denn die Hydrierung soll bis auf einen geringen, tolerierbaren Restgehalt der Acetylene und Diene ohne Verlust an Ethylen bzw. Propylen erfolgen.

Dazu wurden promotierte oder unpromotierte Edelmetallkatalysatoren, meist Palladium-haltig auf Al₂O₃-Trägern mit geringer Oberfläche und mit Aktivkomponentengehalten von 0,01 bis 1 Gew.-% entwickelt.

In Chem. Abstracts, Vol. 82, 169 991 (JP-B-80/047 015) wird die Verwendung von 0,05 Gew.-% Palladium auf makroporösen Al₂O₃-Trägern mit einer BET-Oberfläche von 0,1 bis 2 m²/g als vorteilhaft beschrieben. Der Acetylengehalt kann mit diesen Katalysatoren bis auf 10 ppm gesenkt werden.

Aus der US-A-4 493 906 ist die Verwendung von feinverteiltem Kupfer auf gamma-Al₂O₃, das bis zu 35 % alpha-Al₂O₃ enthält, bekannt. Das verwendete gamma-Al₂O₃ hat eine Oberfläche von 68 bis 350 m²/g und 40 bis 98 % der Poren weisen einen Porendurchmesser zwischen 4 und 12 nm, 2 und 25 % weisen einen Porendurchmesser von 100 bis 1000 nm auf. Auch bimetallische Katalysatoren sind zur selektiven Acetylenhydrierung bekannt.

Aus EP-A-64 301 ist die Verwendung von Pd/Ag-Katalysatoren mit 0,01 bis 0,025 Gew.-% Pd und 2 bis 6 mal soviel Ag bezogen auf Palladium auf alpha-Al₂O₃ mit einer Oberfläche von 3 bis 7 m²/g und einem Porendurchmesser von 685 bis 2270 Å bekannt. Diese Katalysatoren zeichnen sich durch eine geringere CO-Empfindlichkeit und längere Standzeiten aus.

Pd/Au-Katalysatoren mit 0,03 bis 1 Gew.-% Palladium und 0,003 bis 0,3 Gew.-% Gold auf Al₂O₃-Träger und einer Oberfläche von 1 bis 100 m²/g sind aus EP-A-89 252 bekannt. Es wird die Vergiftungsunempfindlichkeit und die geringe Tendenz Oligomere in Anwesenheit von Acetylen zu bilden, beschrieben.

Aus der US-A-4 577 047 sind Pd/Cr-Katalysatoren mit 0,01 bis 0,5 Gew.-% Palladium im Molverhältnis von Palladium zum Chrom von 0,5:1 bis 2:1 auf Al₂O₃-Trägern mit geringer Oberfläche von kleiner 5 m²/g bekannt.

Aus US-A-4 906 800 ist bekannt, daß die Selektivität eines Lindlar-Katalysators (Pd/Pb/CaCO₃) bei Temperaturen größer 100°C durch eine bestimmte Vorbehandlung, die eine Folge verschiedener Oxidations- und Reduktionsschritte beinhaltet, verbessert werden kann.

Ferner ist allgemein bekannt, daß neben Al₂O₃ auch andere Träger für edelmetallhaltige Katalysatoren zur selektiven Acetylenhydrierung möglich sind.

Aus der US-A-4 839 329 ist die Verwendung von Palladium auf TiO₂ mit einer Oberfläche von 30 bis 60 m²/g und aus DE-A-12 84 403 die Verwendung von Pd/Zn auf SiO₂ als Katalysator für die selektive Acetylenhydrierung bekannt.

Es ist allgemein bekannt, dem Reaktionsgemisch bei der Hydrierung von Acetylen Kohlenmonoxid beizumischen, um die Selektivität des Katalysators zu erhöhen. Der Nachteil dieser Verfahren besteht darin, daß die Selektivitätssteigernde Wirkung des Kohlenmonoxids stark temperaturabhängig ist. Große Temperaturgradienten im Katalysatorbett haben daher eine Verschlechterung der Selektivität zur Folge. Außerdem begünstigen die höheren notwendigen Arbeitstemperaturen bei der Zudotierung von CO eine vermehrte Bildung von unerwünschten Polymeren (Grünöl).

Die zuvor beschriebenen Katalysatoren für die selektive Hydrierung von Acetylenen und Dienen werden im allgemeinen durch Tränken eines inerten Trägers mit einer wäßrigen Lösung eines Palladiumsalz, eines Gemisches eines Palladiumsalzes mit einem Salz des Promotors oder durch sequentielle getrennte Tränkung mit wäßrigen Lösungen der Aktivkomponenten- oder Promotor-haltigen Salze, nachfolgender Trocknung und Calcinierung bei höheren Temperaturen hergestellt.

Die zuvor beschriebenen Katalysatoren haben den Nachteil, daß mit zunehmendem Gehalt an Promotor zwar die Selektivität steigt, die Aktivität jedoch stark abnimmt. Zur Erreichung zufriedenstellender Aktivitäten sind daher hohe Temperaturen notwendig.

Der vorliegenden Erfindung lag daher die Aufgabe zugrunde, den zuvor genannten Nachteilen abzuhelfen, insbesondere Katalysatoren für die selektive Hydrierung von Acetylenen und Dienen in der Gasphase bereitzustellen, die hohe Aktivitäten und Selektivitäten bei niedrigen Betriebstemperaturen erlauben und die Hydrierung von Acetylen im C₂-Strom ohne Moderatoren wie CO möglich machen.

Demgemäß wurden neue und verbesserte Palladium-Trägerkatalysatoren mit einem Palladiumgehalt von 0,001 bis 2 Gew.-%, in dem das Palladium, im wesentlichen in Abwesenheit von Promotor-metallen, in einer Schichtdicke von weniger als 5000 nm als Schale auf dem Träger vorliegt, gefunden, welche dadurch gekennzeichnet sind, daß man das Palladium als Sol durch Tränkung auf einen Träger oder durch Aufsprühen auf einen beheizten Träger aufbringt sowie deren Einsatz zur Hydrierung von Acetylenen und Dienen.

Die erfindungsgemäßen Palladium-Trägerkatalysatoren haben einen Gehalt an Palladium von 0,001 bis 2 Gew.-%, bevorzugt 0,005 bis 0,5 Gew.-%, besonders bevorzugt 0,005 bis 0,02 Gew.-% und sind im wesentlichen frei von Promotormetallen, d.h. die Katalysatoren enthalten 0 bis 1 Gew.-%, bevorzugt 0 bis 0,1 Gew.-%, besonders bevorzugt 0 Gew.-% an Promotormetallen bezogen auf die Menge an Palladium, wovon die im eingesetzten Palladium eventuell enthaltenen Mengen an Fremdstoffen unabhängig ist. Ferner haben die Palladium-Trägerkatalysatoren eine Schichtdicke des Palladiums von weniger als 5000 nm, also 10 bis 4900 nm, bevorzugt 50 bis 4000 nm, besonders bevorzugt 100 bis 3500 nm, als Schale auf dem Träger.

Die erfindungsgemäßen Palladium-Trägerkatalysatoren lassen sich wie folgt herstellen:

Die Herstellung des Katalysators erfolgt durch Besprühen eines heißen Trägers oder durch Tränkung des Trägers mit einem zuvor hergestellten Palladiumsol.

Das Palladiumsol kann ausgehend von Palladiumsalzen, in denen das Palladium in der Oxidationsstufe zwei oder vier vorliegt, hergestellt werden. Es können z.B. wäßrige Lösungen der Chloride, Acetate oder Nitrate des Palladiums verwendet werden, es sind aber auch andere Palladiumsalze möglich, eine Beschränkung hinsichtlich des Anions besteht nicht. Als Reduktionsmittel lassen sich organische Verbindungen wie Ethanol, Methanol, Carbonsäuren und deren Alkalisalze sowie anorganische Verbindungen wie N₂H₄ oder NaBH₄ verwenden. Die Teilchengröße der Metallpartikel im Sol hängt hierbei von der Stärke des eingesetzten Reduktionsmittels und von dem verwendeten Metallsalz ab. Im allgemeinen beobachtet man mit stärkeren Reduktionsmitteln die Bildung von kleineren Metallpartikeln. Die Sole lassen sich durch Zugabe von organischen Polymeren wie Polyaminen, Polyvinyl-pyrrolidon oder Polyacrylaten stabilisieren. Die Solherstellung kann aber auch nach anderen in der Literatur beschriebenen Vorgehensweisen durchgeführt werden. In der Angew. Chemie, 103 (1991), Seite 1344 bis 1346 ist beispielsweise die Herstellung von stabilen Metallsolen durch Reduktion von Metallsalzen mit (C₈H₁₇)₄N⁺[BEtH₃] beschrieben.

Als Trägersubstanzen kommen anorganische Oxide wie Al₂O₃, SiO₂, ZrO₂ oder TiO₂ und Gemische davon in Frage. Besonders bewährt haben sich Al₂O₃ und SiO₂.

Die Aufbringung der Sole auf den Träger kann nach verschiedenen Techniken erfolgen, die die Verteilung der Aktivkomponente beeinflussen. Zur Erzeugung dünner Schalen der Aktivkomponente über den gesamten Strangquerschnitt wird das Sol auf einen indirekt beheizten Träger aufgesprüht. Man geht dabei so vor, daß der Träger in einem drehbaren, beheizbaren Pelletierteller vorgelegt und durch ein Heißluftgebläse auf Temperaturen zwischen 80 und 200°C aufgeheizt wird. Während der Teller gedreht wird, sprüht man das Sol durch eine Zweistoffdüse auf den Träger auf. Durch das Drehen des Tellers wird die Durchmischung der Trägerteilchen, z.B. Stränge oder Splitt gewährleistet. Beim Kontakt mit dem heißen Träger verdampft die Flüssigkeit im Sol und die Aktivkomponente bleibt auf dem Träger zurück. Durch diese Aufbringtechnik entstehen Katalysatoren, in denen die Aktivkomponente in dünnen Schichten kleiner 5 µm auf dem Träger aufgebracht ist. Die Teilchengröße der Edelmetallagglomerate liegt im allgemeinen in derselben Größenordnung wie im Sol. Der Katalysator wird dann bei einer Temperatur, die in der Regel 150°C nicht überschreitet getrocknet.

Eine andere Technik zur Aufbringung der Aktivkomponente besteht darin, den Träger entsprechend seiner vorher ermittelten Wasseraufnahme, die im wesentlichen seinem Porenvolumen entspricht, mit einem Sol zu tränken. Nach Abtropfen des Trägers wird dieser dann bei einer Temperatur, die in der Regel 150°C nicht übersteigt, getrocknet. Derart hergestellte Katalysatoren weisen die Aktivkomponente überraschenderweise ebenfalls in einer sehr dünnen Schicht auf. In diesem Fall liegt die Aktivkomponente jedoch in den von außen zugänglichen Makroporen angereichert vor, während beim Aufsprühen des Sols im wesentlichen eine Gleichverteilung der Aktivkomponente in Mikro- und Makroporen erfolgt. Ein wesentlicher Vorteil der Soltränk- und Solaufsprühtechnik besteht darin, daß die Aktivkomponente nach Aufbringen des Sols auf dem Träger im wesentlichen bereits im reduzierten Zustand vorliegt. Damit entfällt eine Reduktion der Aktivkomponente bei hohen Temperaturen, die im allgemeinen ein Zusammensintern der Aktivkomponente bewirkt und dadurch die katalytische Oberfläche verringert.

Die erfindungsgemäßen Katalysatoren haben den Vorteil, daß sie im Gegensatz zu Katalysatoren, die durch Tränkung des Trägers mit einer Metallsalzlösungen hergestellt wurden, höhere Selektivitäten und Aktivitäten in der Hydrierung von Acetylenen und Dienen in Gegenwart von großen Mengen der entsprechenden monoungesättigten Verbindungen aufweisen. Speziell bei der C₂-Hydrierung haben sie den Vorteil hoher Selektivität ohne Zudotierung von CO, das oft zu verfahrenstechnischen Komplikationen führt. Ein weiterer Vorteil ist, daß keine selektivitätssteigernden, aber desaktivierenden Elemente als Promotoren zugesetzt werden müssen. Schließlich erweist sich als Vorteil, daß die erfindungsgemäß hergestellten Katalysatoren mit SiO₂ und Al₂O₃ als Trägersubstanzen gleichermaßen aktiv und selektiv sind.

Die selektiven Hydrierungen von Acetylenen und Dienen werden in der Regel in der Gasphase bei Drücken von 5 bis 50 bar, bevorzugt 8 bis 40 bar, besonders bevorzugt 10 bis 30 bar, Raum-Geschwindigkeiten von 1000 bis 5000m³/m³_{*}h und Temperaturen von 10 bis 150°C, bevorzugt 20 bis 120°C, besonders bevorzugt 25 bis 90°C durchgeführt. Abhängig vom Gehalt der zu hydrierenden Komponenten im Feedgas ergibt sich die Anzahl der Reaktoren. Für Acetylen- und Diengehalte unter 1 % genügt ein adiabatischer Reaktor, das H₂/Acetylen-Dien-Verhältnis beträgt in der Regel etwa 1,1:1 bis 2:1. Dies ist der bei der Hydrierung von Acetylen der am weitesten verbreitete Fall. Ist der Gehalt an Acetylenen und Dienen höher, wird die Hydrierung in zwei oder mehreren in Serie geschaltenen Reaktoren durchgeführt. In diesem Fall wird in der Regel der Wasserstoff vor jedem Reaktor zugeleitet. Die Hydrierung des C₃-Stroms erfolgt meist in drei hintereinandergeschaltenen Reaktoren, wobei im ersten ein Umsatz von 60 bis 70 % und im zweiten ein Umsatz von 30 bis 40 % erzielt wird. Der dritte Reaktor wird für den Restumsatz benötigt oder dient als Sicherheitsreaktor. Bei Acetylengehalten über 2 % im C₂-Strom wird die Hydrierung üblicherweise in einem isothermen Reaktor und einem oder zwei angeschlossenen adiabatischen Reaktoren durchgeführt.

Vor dem Anfahren des Reaktors sollte in der Regel der Sauerstoff durch einen Inertgasstrom entfernt werden, wobei langsam auf 100 bis 150°C erhitzt wird. Bei dieser Temperatur wird der Edelmetallkatalysator mit H₂ in der Regel reduziert.

Für Performancetests können die Katalysatoren in einer drucklosen Laborapparatur oder in einer Technikumsapparatur unter erhöhten Drücken von 5 bis 50 bar, bevorzugt 10 bis 30 bar eingesetzt werden. Die Eintrittstemperaturen des in die Hydrierzone eintretenden Gasgemisches betragen im allgemeinen 15 bis 120°C, vorzugsweise 25 bis 90°C. Das Volumenverhältnis von Wasserstoff zu den mehrfach ungesättigten Kohlenwasserstoffen beträgt im allgemeinen 0,5:1 bis 2,5:1, bei der C₂-Hydrierung vorzugsweise 1,1:1 bis 2:1, insbesondere 1,2:1 bis 1,8:1 und bei der ersten Stufe der C₃-Hydrierung 0,6:1 bis 0,9:1.

### Beispiele

### Beispiel 1

### Herstellung des Katalysators

Zur Darstellung eines stabilen Pd-Sols wurden 2.165 g Pd(NO₃)₂ und etwa 5g Polyvinylpyrrolidon in 1000 ml eines 1:1 Gemisches aus Ethanol und Wasser gelöst. Die Lösung wurde 1/2 h bei Raumtemperatur gerührt und anschließend 4 h zum Rückfluß erhitzt. Nach Abkühlen wurde ein Palladiumsol mit einem Gehalt von 1g Pd/l erhalten. 180 ml dieses Sols wurden mit 120 ml dest. Wasser auf 300 ml verdünnt. 300 g eines Al₂O₃-Trägers mit einem Gesamtporenvolumen von 0.849 cm³/g, einer BET-Oberfläche von 357 m²/g und einer Wasseraufnahme von 1 ml/g wurden mit dem verdünnten Sol 1/2 h lang getränkt und nach Abtropfen 16 h bei 120°C getrocknet. Nach dieser Trockenzeit enthielt der Katalysator laut Analyse 0.07 Gew.-% Palladium bezogen auf den Trägerkatalysator.

Der erhaltenene Katalysator wurde für die Selektivhydrierung von Acetylen getestet. Hierzu wurde bei 27°C und 20 bar Druck ein Gasgemisch aus etwa 99 % Ethylen und 1 % Acetylen in einem Festbettreaktor über 29.1 g des oben beschriebenen Katalysators geleitet. Das H₂/Acetylen Verhältnis betrug 1.12, die Belastung 3000 h⁻¹. Unter diesen Bedingungen wurde ein vollständiger Acetylenumsatz bei einer Selektivität für Acetylen von 66.0 % erzielt.

### Beispiel 2

Der nach Beispiel 1 hergestellte Katalysator zeigt auch bei druckloser Fahrweise eine sehr hohe Selektivität zu Ethylen. Hierzu wurde bei 73°C und 1 bar Druck ein Gasgemisch aus etwa 99 % Ethylen und 1 % Acetylen in einem Festbettreaktor über 14.0 g des oben beschriebenen Katalysators geleitet. Das H₂/Acetylen-Verhältnis betrug 1.11, die Belastung 3000 h⁻¹. Unter diesen Bedingungen wurde ein Acetylenumsatz von 94.7 % bei einer Selektivität für Acetylen von 76.0 % erzielt.

### Beispiel 3

### Herstellung des Katalysators und selektive Acetylenhydrierung

1.603 g einer 11 %igen Pd(NO₃)₂-Lösung wurden mit 1170 ml bidestilliertem H₂O gemischt und die entstandene Lösung mit 5 g Polyvinylpyrrolidon versetzt. Zu dieser Lösung wurden 25 ml einer 0.8 %igen Hydrazinhydratlösung gegeben. Es wurde 1/2 h bei Raumtemperatur gerührt. Anschließend wurde die Lösung zum Rückfluß erhitzt und 4 h bei dieser Temperatur gerührt. Nach Abkühlen erhielt man ein stabiles Sol. 120 ml dieses Sols wurden mit bidestilliertem Wasser auf 11 verdünnt. Das verdünnte Sol wurde im beheizten Pelletierteller auf 90 g des in Beispiel 1 beschriebenen Al₂O₃-Trägers gesprüht. Der Katalysator wurde 16 h bei 120°C getrocknet. Laut Analyse enthielt dieser Katalysator 0.009 Gew.-% Pd, bezogen auf den Träger.

Zur Acetylenhydrierung wurde verfahren wie in Beispiel 2 beschrieben, die Gaseintrittstemperatur betrug jedoch 87°C und das H₂/C₂H₂-Verhältnis 1.82. Es wurde ein Acetylenumsatz von 90.5 % bei einer Selektivität für Acetylen von 64.0 % erzielt.

### Beispiel 4

### Herstellung des Katalysators und selektive C₂-Hydrierung

1.365 g einer 11 %igen Pd(NO₃)₂-Lösung wurde mit 2.25 l destilliertem H₂O gemischt und die Lösung mit 1.5 g Polyvinylpyrrolidon versetzt. Zu dieser Lösung wurden 750 ml Ethanol gegeben und die Lösung 4 h zum Rückfluß erhitzt. Nach Abkühlen wurde ein stabiles Sol erhalten. 400 ml dieses Sols wurden auf 11 verdünnt. Das verdünnte Sol wurde im beheizten Pelletierteller auf 100 g eines SiO₂-Trägers mit einer Gesamtporenfläche von 0.95 cm³/g, einer BET-Oberfläche von 136 m²/g und einer Wasseraufnahme von 1 ml/g gesprüht. Anschließend wurde der Katalysator 16 h bei 120°C getrocknet.

Zur Acetylenhydrierung wurde verfahren wie in Beispiel 2 beschrieben, die Gaseintrittstemperatur betrug jedoch 82°C und das H₂/C₂H₂-Verhältnis 1.74. Es wurde ein Acetylenumsatz von 85.1 % bei einer Selektivität für Acetylen von 66.8 % erzielt.

Bei einer Gaseintrittstemperatur von 92°C und einem H₂/C₂H₂-Verhältnis von 1.72 wurde ein Acetylenumsatz von 98.2 % bei einer Selektivität für Acetylen von 46.4 % erzielt.

### Beispiel 5

114 ml einer 7.4 x 10⁻³ M PdCl₂-Lösung wurden mit 1304 ml bidestilliertem H₂O gemischt. Zu dieser Lösung wurden 5 g Polyvinylpyrrolidon und 180 ml einer 0.034 M Natriumcitratlösung gegeben. Die Reaktionslösung wurde 4 h am Rückfluß erhitzt. Nach Abkühlen erhielt man ein stabiles Sol. 1110 ml dieses Sols wurden im beheizten Pelletierteller auf 92.5 g des in Beispiel 3 beschriebenen SiO₂-Trägers gesprüht. Anschließend wurde der Katalysator 16 h bei 120°C getrocknet. Der Palladiumgehalt dieses Katalysators betrug laut Analyse 0.06 Gew.-% Pd.

Zur Acetylenhydrierung wurde verfahren wie in Beispiel 2 beschrieben, die Gaseintrittstemperatur betrug jedoch 87°C und das H₂/C₂H₂-Verhältnis 1.76. Es wurde ein Acetylenumsatz von 90.5 % bei einer Selektivität für Acetylen von 54.3 % erzielt.

### Beispiel 6

Zu 0.909 g einer 11 Gew.-% igen Pd(NO₃)₂-Lösung wurden 760 ml bidestilliertes H₂O und 0.5 g Polyvinylamin gegeben. Diese Lösung wurde mit 240 ml einer 0.034 M Natriumformiatlösung versetzt und 4 h am Rückfluß erhitzt. Nach Abkühlen erhielt man ein stabiles Sol. 200 ml dieses Sols wurden auf 1 l verdünnt. Das verdünnte Sol wurde im beheizten Pelletierteller auf 113 g des in Beispiel 1 beschriebenen Al₂O₃-Trägers gesprüht. Der Katalysator wurde anschließend 16 h bei 120°C getrocknet.

Zur Acetylenhydrierung wurde verfahren wie in Beispiel 2 beschrieben, die Gaseintrittstemperatur betrug jedoch 97°C und das H₂/C₂H₂-Verhältnis 1.78. Es wurde ein Acetylenumsatz von 92.1 % bei einer Selektivität für Acetylen von 49.2 % erzielt.

### Beispiel 7

Zu 27.27 g einer 11 Gew.-% igen Lösung von Pd(NO₃)₂ wurden 495 ml bidestilliertes H₂O, und 5 g Polyvinylpyrrolidon gegeben. Diese Lösung wurde mit 495 ml Ethanol versetzt und 4 h am Rückfluß gerührt. Nach Abkühlen erhielt man ein stabiles Sol. 30 ml dieses Sols wurden mit destilliertem H₂O auf 300 ml verdünnt. 100 g des in Beispiel 3 beschriebenen SiO₂-Trägers wurden 1/2 h mit dem verdünnten Sol getränkt und nach dem Abtropfen 16 h bei 120°C getrocknet. Der Palladiumgehalt des Katalysators betrug laut Analyse 0.035 Gew.-% Pd, bezogen auf den Träger.

Zur Acetylenhydrierung wurde verfahren wie in Beispiel 1 beschrieben, die Gaseintrittstemperatur betrug jedoch 63°C und das H₂/C₂H₂-Verhältnis 1.76. Es wurde ein Acetylenumsatz von 82.0 % bei einer Selektivität für Acetylen von 52.7 % erzielt.

### Beispiel 8

Zur Darstellung eines stabilen Pd-Sols wurden 0.373 g Pd-acetat und etwa 5g Polyvinylpyrrolidon in 1170 ml Wasser gelöst. Zu dieser Lösung wurden unter starkem Rühren tropfenweise 25 ml einer 0.8 % Hydrazinhydrat-Lösung gegeben. Die Reaktionslösung wurde anschließend 2h zum Rückfluß erhitzt. Nach Abkühlen wurde ein stabiles Palladiumsol mit einem Gehalt von 0.15 g Pd/l erhalten. 400 ml dieses Sols wurden im beheizten Pelletierteller auf 100 g eines SiO₂-Trägers mit einer Gesamtporenflächen von 0.95 cm³/g einer BET-Oberfläche von 136 m²/g und einer Wasseraufnahme von 1 ml/g gesprüht. Anschließend wurde der Katalysator 16 h bei 120°C getrocknet.

### Beispiel 9

### C₃-Hydrierung Gasphase

Der im vorigen Beispiel hergestellte Katalysator wurde einem Test für die Gasphasenhydrierung von Methylacetylen und Propadien im C₃-Strom ex Steamcracker unterzogen. Die Verfahrensbedingungen wurden den Bedingungen einer ersten Stufe der meist dreistufigen Methylacetylen/Propadien-Hydrierung im C₃-Strom angelegt. In einen adiabatisch-betriebenen Rohrreaktor (Durchmesser 20 mm) wurden 70 ml des nach Beispiel 8 hergestellten Katalysators eingebaut. Nach Spülen mit Stickstoff und einer Wasserstoffbehandlung bei 120°C wurden 650 l/h eines Gasgemisches mit der Zusammensetzung 5,2 % Propan, 1,7 % Propadien und 2,4 % Methylacetylen in Propylen und 21 l/h Wasserstoff bei einer Eintrittstemperatur von 60°C und einem Druck von 10 bar über den Katalysator geführt. Der Reaktions-austrag bestand aus 6,4 % Propan, 0,42 % Propadien, 0,31 % Methylacetylen in Propylen. Dies entspricht einer Selektivität zu Propylen von 79 % bei einem Umsatz von 70 %.

## Patentansprüche

1. Palladium-Trägerkatalysatoren mit einem Palladiumgehalt von 0,001 bis 2 Gew.-%, in dem das Palladium, im wesentlichen in Abwesenheit von Promotormetallen, in einer Schichtdicke von weniger als 5000 nm als Schale auf dem Träger vorliegt, dadurch gekennzeichnet, daß man das Palladium als Sol durch Tränkung auf einen Träger oder durch Aufsprühen auf einen beheizten Träger aufbringt.

2. Palladium-Trägerkatalysatoren nach Anspruch 1, dadurch gekennzeichnet, daß man das Palladium durch Tränkung des Trägers mit dem Edelmetallsol aufbringt.

3. Palladium-Trägerkatalysatoren nach Anspruch 1, dadurch gekennzeichnet, daß man als Träger anorganische Oxide einsetzt.

4. Palladium-Trägerkatalysatoren nach Anspruch 1, dadurch gekennzeichnet, daß man als Träger Al₂O₃, SiO₂, ZrO₂, TiO₂ oder deren Gemische einsetzt.

5. Palladium-Trägerkatalysatoren nach Anspruch 1, dadurch gekennzeichnet, daß man als Träger Al₂O₃, SiO₂ oder deren Gemische einsetzt.

## Claims

1. A supported palladium catalyst having a palladium content of from 0.001 to 2 % by weight in which the palladium, essentially in the absence of promoter metals, is present as a shell having a thickness of less than 5000 nm on the support, wherein the palladium is applied as sol to a support by impregnation or by spraying onto a heated support.

2. A supported palladium catalyst as claimed in claim 1, wherein the palladium is applied by impregnating the support with the noble metal sol.

3. A supported palladium catalyst as claimed in claim 1, wherein the support used comprises inorganic oxides.

4. A supported palladium catalyst as claimed in claim 1, wherein the support used is Al₂O₃, SiO₂, ZrO₂, TiO₂ or a mixture thereof.

5. A supported palladium catalyst as claimed in claim 1, wherein the support used is Al₂O₃, SiO₂ or a mixture thereof.

## Revendications

1. Catalyseurs supportés au palladium ayant une teneur en palladium de 0,001 à 2 % en poids, dans lesquels le palladium se trouve sur le support sous la forme d'une coquille en une couche épaisse de moins de 5000 nm, en l'absence de métal promoteur, pour l'essentiel, caractérisés en ce que l'on dépose le palladium sous forme de sol par imprégnation sur un support ou par pulvérisation sur un support chauffé.

2. Catalyseurs supportés au palladium selon la revendication 1, caractérisés en ce que l'on dépose le palladium par imprégnation du support avec le sol de métal précieux.

3. Catalyseurs supportés au palladium selon la revendication 1, caractérisés en ce que l'on utilise un oxyde minéral en tant que support.

4. Catalyseurs supportés au palladium selon la revendication 1, caractérisés en ce que l'on utilise Al₂O₃, SiO₂, ZrO₂, TiO₂ ou leurs mélanges en tant que support.

5. Catalyseurs supportés au palladium selon la revendication 1, caractérisés en ce que l'on utilise Al₂O₃, SiO₂ ou leurs mélanges en tant que support.
